(19) **Europäisches Patentamt / European Patent Office / Office européen des brevets**

(11) **EP 3 905 254 B1**

(12) **EUROPEAN PATENT SPECIFICATION**

(45) Date of publication and mention of the grant of the patent:
**10.12.2025 Bulletin 2025/50**

(21) Application number: **21171667.5**

(22) Date of filing: **30.04.2021**

(51) International Patent Classification (IPC):
**G16C 20/30** (2019.01)    **G16C 20/90** (2019.01)
G16C 20/10 (2019.01)    G16C 60/00 (2019.01)

(52) Cooperative Patent Classification (CPC):
**G16C 20/30; G16C 20/90;** G16C 20/10;
G16C 60/00

(54) **VOLATILE LIQUID CHEMICAL COMPOUND PHYSICAL PARAMETER DATABASE CONSTRUCTION METHOD, AND PREDICTION MODEL**

VERFAHREN ZUM AUFBAU EINER DATENBANK DER PHYSIKALISCHEN PARAMETER VON FLÜCHTIGEN FLÜSSIGEN CHEMISCHEN VERBINDUNGEN, UND VORHERSAGEMODELL

PROCÉDÉ DE CONSTRUCTION D'UNE BASE DE DONNÉES DE PARAMÈTRES PHYSIQUES DE COMPOSÉS CHIMIQUES VOLATILS ET MODÈLE DE PRÉDICTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2020 EP 20172487**

(43) Date of publication of application:
**03.11.2021 Bulletin 2021/44**

(73) Proprietor: **Firmenich SA**
**1242 Satigny (CH)**

(72) Inventors:
- **VUILLEUMIER, Christine**
  **1242 Satigny (CH)**
- **AYMARD, Laurence**
  **1242 Satigny (CH)**
- **DE SAINT LAUMER, Jean-Yves**
  **1242 Satigny (CH)**
- **FITZGERALD, Sanja**
  **1242 Satigny (CH)**
- **GODIN, Guillaume**
  **1242 Satigny (CH)**

(74) Representative: **dsm-firmenich IP**
**DSM-Firmenich AG**
**Wurmisweg 576**
**4303 Kaiseraugst (CH)**

(56) References cited:
**WO-A1-2019/238680**    **SU-A1- 1 013 822**

- **EVGENIYA G. ORLOVA ET AL: "Evaporation Rate of a Liquid Layer Streamlined by Gas Flow in Minichannel", MATEC WEB OF CONFERENCES, vol. 91, 20 December 2016 (2016-12-20), XP055744540, DOI: 10.1051/matecconf/20179101028**
- **PENPAN SAIYASOMBATI ET AL: "Disposition of Benzyl Alcohol After Topical Application to Human Skin in vitro", JOURNAL OF PHARMACEUTICAL SCIENCES, vol. 92, no. 10, 30 July 2003 (2003-07-30), US, pages 2128 - 2139, XP055743963, ISSN: 0022-3549, DOI: 10.1002/jps.10467**
- **G.B. KASTING ET AL: "A physico-chemical properties based model for estimating evaporation and absorption rates of perfumes from skin", INTERNATIONAL JOURNAL OF COSMETIC SCIENCE., vol. 23, no. 1, 1 February 2001 (2001-02-01), NL, pages 49 - 58, XP055743951, ISSN: 0142-5463, DOI: 10.1046/j.1467-2494.2001.00079.x**
- **SCHWARZENBACH R ET AL: "MODELS TO ASSESS PERFUME DIFFUSION FROM SKIN", INTERNATIONAL JOURNAL OF COSMETIC SCIENCE, KLUWER ACADEMIC PUBLISHERS, DORDRECHT, NL, vol. 23, no. 2, 1 January 2001 (2001-01-01), pages 85 - 98, XP009058568, ISSN: 0142-5463, DOI: 10.1046/J.1467-2494.2001.00067.X**

EP 3 905 254 B1

**(Cont. next page)**

- RAFAEL N. ALMEIDA ET AL: "Evaporation and Permeation of Fragrance Applied to the Skin", INDUSTRIAL & ENGINEERING CHEMISTRY RESEARCH, vol. 58, no. 22, 16 May 2019 (2019-05-16), pages 9644 - 9650, XP055743952, ISSN: 0888-5885, DOI: 10.1021/acs.iecr.9b01004
- P. SAIYASOMBATI ET AL: "Two-stage kinetic analysis of fragrance evaporation and absorption from skin", INTERNATIONAL JOURNAL OF COSMETIC SCIENCE., vol. 25, no. 5, 1 October 2003 (2003-10-01), NL, pages 235 - 243, XP055741607, ISSN: 0142-5463, DOI: 10.1046/j.1467-2494.2003.00193.x

**Description**

TECHNICAL FIELD OF THE INVENTION

The present invention relates to a volatile liquid chemical compound physical parameter database construction method, a fragrance physical composition evolution prediction method and the corresponding systems. It applies, in particular, to the fields of fragrance design, perfumery, fine fragrance perfumery and flavor design.

BACKGROUND OF THE INVENTION

Fragrance design can be defined as the selection of at least one fragrant ingredient to form a composition intended to provide a targeted fragrance. Fragrance design is most notably known in the field of perfumery and is performed by perfumers

The evaluation of a fragrance is based on performance metrics and fragrance hedonics. Several metrics are used today, such as the detectability of the fragrance by a human nose for example. While such metrics can be measured, few can accurately be predicted.

One such unsatisfying metric prediction is the prediction of a fragrance evaporation over time.

Fragrance evaporation defines the fragrance's lastingness, variability in olfactive character and strength over time. To predict fragrance evaporation over time there are many classical approaches described in the literature, based mainly on Fick's law or Raoul's law, based on diffusion equations, mass transfer, equilibrium partial vapor pressures etc.

An example of such an approach is disclosed in patent application n° WO2019/238680 filed by the company Givaudan. This patent application aims at "a computer-implemented method of predicting the temporal fragrance profile of a fragrance composition comprising a plurality of fragrance ingredients, the method comprising using a processor to: retrieve a diffusion measure of how fast each fragrance ingredient diffuses into a headspace; form groups of fragrance ingredients having the same or a similar diffusion measure; determine the olfactive contribution of each fragrance ingredient; calculate the total olfactive contribution of a group of fragrance ingredients as the sum of the olfactive contributions of all fragrance ingredients forming said group; and using a graphical user interface (GUI) to display the total olfactive contribution of each group of fragrance ingredients in the order of their respective diffusion measures to visualize the temporal fragrance profile of the fragrance composition."

In such a patent application, the model is based on diffusion of fragrant compounds and these diffusion indexes are used in a fragrance evolution prediction method.

However, such models have several key performance drawbacks:

- they are largely concerned with a prediction of evaporation of a fragrance from a liquid bulk (fragrance bottle) which is not representative of a fragrance being stripped from a surface (i.e. skin),
- they are based on complex theoretical laws (Stefan tube evaporation theory) requiring an important computation time to provide results and are thus impossible to use in real-time,
- they are based on the measurement of a compound's vapor pressure, which provides unsatisfying results at room temperature and
- they consider fragrance compositions as a unitary whole, providing no consideration for the internal evolution of the composition over time - the strongest ingredient is considered to be the only perceivable ingredient.

At this stage, it is key to understand that the way evaporation is measured in current models is not representative of the way a fragrance is transferred into the air.

Furthermore, it is important to note that measuring a compound's vapor pressure at room temperature is extremely difficult and that such approaches require the use of very sensitive pressure sensors located around a measured compound quantity. These sensitive pressure sensors are such that the measurement cannot be performed at room temperature and require the temperature to be increased by several orders of magnitude. From gathering the vapor pressure values at higher temperatures, the vapor pressure at room temperature is extrapolated. Hence, such approaches are inaccurate.

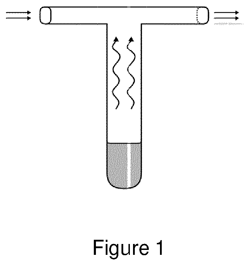

Evaporation is defined as the "evaporation of water, by vaporization, from aqueous solution of nonvolatile substances". The way evaporation is measured in contemporary systems is shown in figure 1. Such systems use a Stefan tube in which a composition is deposited and through which an airflow flows, allowing for the measurement downstream of the airflow of the quantity of the composition evaporated. In such a system, the airflow is kept away from the surface of the liquid and the surface to depth ratio of the liquid is not representative of a spread on a surface (i.e. skin).

Aside from the transfer of fragrance into an airflow, no predictive performance metrics making use of this information to estimate other key performance metrics are available. Finally, current fragrance performance models are based on the simulation of a vapor pressure value to determine an evaporated quantity of a compound. However, such models are inaccurate at room temperature as they cannot be confirmed empirically.

There are currently no satisfactory system allowing for the delivery of predictive fragrance performance metrics in a real time manner. This lack of satisfactory system carries with it a loss of time for perfumers who are bound to trial and error approaches and a lack of insight allowing for more predictable perfume designs.

As such, perfume designers currently rely on empirical expertise to design fragrances.

- Patent Application SU 1 013 822 which relates to the experimental determination of the volatility of substances and can be used to study the kinetics of evaporation of liquids, and its results when calculating mass transfer processes and apparatuses associated with evaporation from a covered surface.
- The publication by Penpan Saivasombati et al. titled "Disposition of Benzyl Alcohol After Topical Application to Human Skin in vitro" which presents a detailed test of a first-order kinetic approach to modeling the dissipation of a volatile compound or mixture from the skin surface after topical application, involving both diffusion and evaporation.
- The publication bv G.B. Kasting et al. titles "A physico-chemical properties based model for estimating evaporation and absorption rated of perfumes from skin" describing an improved method to estimate the absorption and evaporation of perfume ingredients from skin, based on their physico-chemical properties.

SUMMARY OF THE INVENTION

The present invention is intended to remedy all or part of these disadvantages.

To this effect, according to a first aspect, the present invention aims at a volatile liquid chemical compound physical parameter database construction method, comprising:

- a step of controlled deposition of a chemical compound in an inert container,
- a step of airflow generation, the airflow being directed in the direction of the deposited chemical compound,
- a step of measurement of a quantity of evaporated chemical compound at different measurement times,
- a step of measurement of relative gas phase concentrations for several initial ingredient concentration in the liquid chemical compound at preset experimental pseudo-equilibrium conditions corresponding to equilibrium conditions,
- a step of evaporation rate calculation depending on the measured evaporated chemical compound quantities measured at pseudo-equilibrium conditions,
- a step of volatility calculation depending on the evaporation rate calculated, a first linear regression function linking evaporation rate to concentration at equilibrium conditions and a second linear regression function that converts pseudo-equilibrium evaporation rates into volatility and
- a step of storing, in a database, the calculated evaporation rate and the volatility calculated.

Thanks to these dispositions, the database construction method allows for the accurate measurement of the evaporation rates and volatility of chemical compounds. Preferably, those chemical compounds are chosen to be unitary in order to provide a unitary chemical compound physical parameter database construction method. In the case of fragrance design, these evaporation rate and volatility data allow for the fragrance behavior prediction over time.

In such an approach, evaporation rate and volatility are linked as opposed to the prior art linking vapor pressure to volatility. Evaporation rates provide accurate results as opposed to vapor pressures which are very difficult to measure reliably at room temperature in particular.

In particular embodiments, the method object of the present invention further comprises:

- a step of computing at least one gas phase concentration of a chemical compound for a given volatility of chemical compound,
- a step of measurement of the psychophysical intensity of a chemical compound for at least one said gas phase concentration,
- a step of modeling of a mathematical formula of psychophysical intensity as a function of gas phase concentration based on at least two of the measured gas phase concentration values and
- a step of recording, in a database, the psychophysical intensity formula modeled parameters.

These provisions allow for the association of perceived intensity of the fragrance of a chemical compound with the concentration of said chemical compound in the headspace of a user.

In the case of fragrance design, these provisions allow for the fragrance perceived intensity prediction over time.

In particular embodiments, the method object of the present invention further comprises a plurality of steps of controlled deposition of a chemical compound at different temperature, the evaporation rate being calculated for each said temperature and stored during the step of storing.

In the case of fragrance design, these evaporation rate and volatility data allow for the fragrance behavior prediction over time for a given number of temperatures that can be representative of temperatures at which a fragrance is intended to be used. Such data allows for a more accurate fragrance performance behavior prediction.

According to a second aspect, the present invention aims at a fragrance physical composition evolution prediction method to provide predictive, real-time, fragrance performance metrics, comprising:

- a step of selecting at least one chemical compound identifier in a computerized interface,

- a step of inputting, for each selected chemical compound, a quantity of said chemical compound,
- a step of modeling a deposition of the quantity of each selected chemical compound on a virtual surface,
- a step of simulating, by a computing system, for each modeled deposited chemical compound, the stripped quantity of said chemical compound in an airflow for at least two different times as a function of:

  - the quantity of each said chemical compound,
  - a first value representative of a virtual surface size of the deposited chemical compound,
  - a second value representative of a virtual airflow directed at the deposited chemical compound configured to virtually strip the chemical compound from the surface,
  - a third value representative of an activity coefficient of each said chemical compound and
  - a volatility associated to said chemical compound stored in a database constructed according to the database construction method object of the present invention and

- a step of displaying, for each chemical compound, of an indicator representative of the computed evaporated mass of said compound over time.

Thanks to these dispositions, users of the system can accurately model the behavior of a composition over time in terms of remaining fragrance on the modeled surface or in gas phase. Such a system is much more accurate than the current Stefan tube models which are not representative of a composition dispersion over a substrate.

Such provisions further allow to:

- speed up a composition (such as perfume) creation process,
- optimizing the composition performance,
- reformulating the composition performance easily,
- provide a new understanding to perfumers on their own formulas and
- compare formulas performance (Long lastingness, or olfactive profile for example).

Furthermore, such modeling is representative of conditions of fragrance wearing and can assess how real consumers will perceive a fragrance during its wearing, not what will happen with a fragrance in an open bottle.

In particular embodiments, the method object of the present invention further comprises a step of computing a gas phase concentration of the virtually stripped chemical compound as a function of the stripped quantity computed, the step of displaying being configured to display the gas phase concentration computed.

These provisions allow for the accurate prediction of the amount of each composition ingredient is in a gaseous state, ingredients in such states having the capacity to be sensed by the intended composition targets.

In particular embodiments, the method object of the present invention further comprises a step of computing a psychophysical intensity of each selected chemical compound as a function of the gas phase concentration computed, the step of displaying being configured to display the psychophysical intensity computed over time.

These provisions allow for the accurate prediction of the perceived intensity of the composition as a whole and for each compound taken separately. This allows for a much finer prediction than current bulk models in which only the most fragrant compound is considered representative of the compositions as a whole.

In particular embodiments, at least two chemical compounds are selected, the method further comprising a step of computing a global psychophysical intensity comprising:

- a first step of matching a value for concentration for each compound against the corresponding dose-response curve to provide a perceived intensity value for that compound,
- a second step of matching each said perceived intensity value against a dummy dose-response curve to provide an artificial compound concentration value,
- a step of addition of each artificial compound concentration value to form a virtual concentration value and
- a fourth step of matching the virtual concentration value against the dummy dose-response curve to provide a total perceived intensity value for the composition.

the step of displaying being configured to display the global psychophysical intensity computed over time.

These provisions allow for the accurate prediction of the perceived intensity of the composition as a whole and for each compound taken separately. This allows for a much finer prediction than current bulk models in which only the most fragrant compound is considered representative of the compositions as a whole.

In particular embodiments, at least two chemical compounds are selected, the method further comprising a step of computing a psychophysical intensity linearity of the composition of said at least two compounds based on the computed psychophysical intensity of each selected chemical compound over time, the step of displaying being configured to display the psychophysical intensity linearity of the composition of said at least two compounds.

These provisions allow for the accurate prediction of the evolution of the perceived intensity of the composition as a whole and for each compound taken separately. This allows for a much finer prediction than current bulk models in which only the most fragrant compound is considered representative of the compositions as a whole.

In particular embodiments, the method object of the present invention further comprises a step of chemical compound identifier selection, said chemical compound identifier being selected if the psychophysical intensity at a given time is below a determined value and a step of display of said chemical compound identifier.

These provisions allow for the identification of compounds that provide an insufficient contribution at a given time post-dispersion on the substrate. This, in turns, allow for the correction of the composition by removal of said compounds or increase in the initial quantity of said compounds.

In particular embodiments, at least two chemical compounds are selected to form a composition, the method further comprising a step of computing of the composition evolution over time as a function of the stripped quantity calculated over time.

These provisions allow for the measurement of inter-compound interaction as a factor of the evolution of the composition over time.

In particular embodiments, the method object of the present invention further comprises a step of construction of the liquid chemical compound physical parameter database storing the volatility associated to said chemical compound by carrying out the method subject of the present invention.

Such embodiments provide similar advantages to the liquid chemical compound physical parameter database construction method object of the present invention.

According to a third aspect, the present invention aims at a liquid chemical compound physical parameter database construction system, comprising:

- a means of controlled deposition of a chemical compound in an inert container,
- a means of airflow generation, the airflow being directed in the direction of the deposited chemical compound,
- a means of measurement of a quantity of evaporated chemical compound at different measurement times,
- a means of measurement of relative gas phase concentrations for several initial ingredient concentration in the liquid chemical compound at preset experimental pseudo-equilibrium conditions corresponding to equilibrium conditions,
- a means of evaporation rate calculation depending on the measured evaporated chemical compound quantities measured at pseudo-equilibrium conditions,
- a means of volatility calculation depending on the maximum evaporation rate calculated, a first linear regression function linking evaporation rate to concentration at equilibrium conditions and a second linear regression function that converts pseudo-equilibrium evaporation rates into volatility and

- a means of storing, in a database, the calculated evaporation rate and the volatility calculated.

Such provisions provide similar advantages to the liquid chemical compound physical parameter database construction method object of the present invention.

According to a fourth aspect, the present invention aims at a fragrance physical parameter evolution prediction system to provide predictive, real-time, fragrance performance metrics, comprising:

- a means of selecting at least one chemical compound identifier in a computerized interface,
- a means of inputting, for each selected chemical compound, a quantity of said chemical compound,
- a means of modeling a deposition of the quantity of each selected chemical compound on a virtual surface,
- a means of simulating, by a computing system, for each modeled deposited chemical compound, the stripped quantity of said chemical compound in an airflow for at least two different times as a function of:

  - the quantity of each said chemical compound,
  - a first value representative of a virtual surface size of the deposited chemical compound,
  - a second value representative of a virtual airflow directed at the deposited chemical compound configured to virtually strip the chemical compound from the surface,
  - a third value representative of an activity coefficient of each said chemical compound and
  - a volatility associated to said chemical compound stored in a database constructed according to the database construction method object of the present invention and

- a means of displaying, for each chemical compound, of an indicator representative of the computed evaporated mass of said compound over time.

Such provisions provide similar advantages to the fragrance physical parameter evolution prediction method object of the present invention.

BRIEF DESCRIPTION OF THE DRAWINGS

Other advantages, purposes and particular characteristics of the invention shall be apparent from the following non-exhaustive description of at least one particular method or system which is the subject of this invention, in relation to the drawings annexed hereto, in which:

[Figure 1] represents, schematically, a Stefan tube used for current evaporation models,
[Figure 2] represents, schematically and in the form of a flowchart, a particular succession of steps of the database construction method, which is the object of the present invention,
[Figure 3] represents, schematically and in the form of a flowchart, a particular succession of steps of the prediction method, which is the object of the present invention,
[Figure 4] represents, schematically, a particular embodiment of a system capable of implementing the database construction method, which is the object of the present invention,

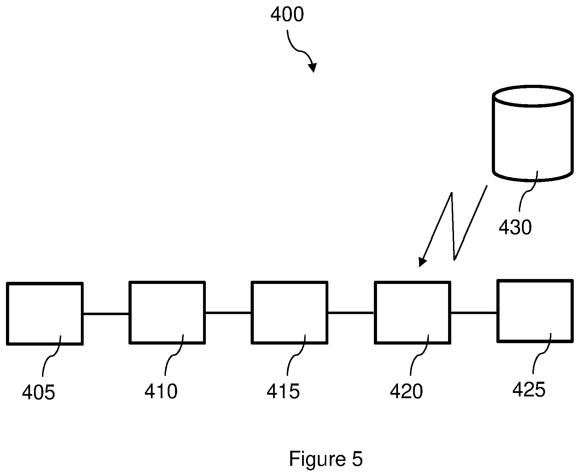

[Figure 5] represents, schematically, a particular embodiment of a system capable of implementing the prediction method which is the object of the present invention and
[Figure 6] represents, schematically, the result of a mathematical formula relating psychophysical perceived intensity of a chemical compound with the gas phase concentration of said chemical compound.

DETAILED DESCRIPTION OF THE INVENTION

This description is not exhaustive, as each feature of one embodiment may be combined with any other feature of any other embodiment in an advantageous manner.
It should be noted at this point that the figures are not to scale.
In the context of this invention, a "compound" designates a molecule, a mixture of isomers, a polymer, an ingredient or a solvent.
It should be noted here that volatility itself has no defined general thermodynamic quantity or value, but it is often described using vapor pressures or boiling points (for liquids). High vapor pressures indicate a high volatility, while high boiling points indicate low volatility. Vapor pressures and boiling points are often presented in tables and charts that can be used to compare chemicals of interest.
In the context of this invention, volatility is preferably expressed in units of concentration such as grams of compound per liter of air which corresponds to the maximum concentration that a compound gaseous form can have in equilibrium with its liquid or solid phase in a closed system.
Volatility can be used to measure the strength of an ingredient as a function of gas phase concentration. Said gas phase concentration can define the Odor Detection Threshold - a gas phase conc at which an odor can be detected.
In the context of this invention, a "volatile compound" designates a compound presenting a high vapor pressure at ordinary room temperatures. Such a compound evaporates at temperatures above a minimal temperature threshold representative of a minimal temperature intended for the use of the compound. For

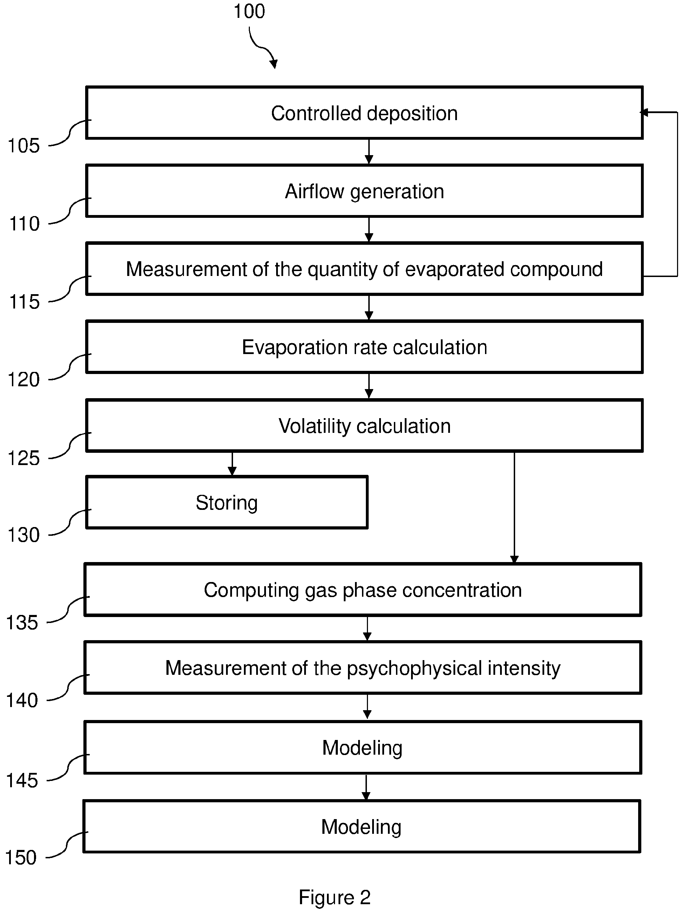

example, if a compound is intended to be used in a fragrance that should be perceived in everyday life, that minimal temperature might be 0°C. In this example, at temperatures above 0°C, the compound forms a vapor called "gas phase". Such a chemical compound can also be defined by the molecular mass of said compound. According to this method of definition, a volatile compound is a compound presenting a molecular mass below 350 Da. Preferably, a volatile compound is a compound presenting a molecular mass below 325 Da. Preferably, a volatile compound is a compound presenting a molecular mass below 300 Da.
It should be noted that the term "inert" is intended as "providing no chemical interactions with a compound of interest". For example, in the context for figure 2, an inert container can be made of aluminum.
Figure 2 shows a particular succession of steps of a method which is the subject of this invention. This volatile liquid chemical compound physical parameter database construction method 100 comprises:

- a step 105 of controlled deposition of a chemical compound in an inert container,
- a step 110 of airflow generation, the airflow being directed in the direction of the deposited chemical compound,
- a step 115 of measurement of a quantity of evaporated chemical compound at different measurement times,
- a step 120 of evaporation rate calculation depending on the measured evaporated chemical compound quantities measured,
- a step 125 of volatility calculation depending on the evaporation rate calculated and
- a step 130 of storing, in a database, the calculated evaporation rate and the volatility calculated.

The step 105 of controlled deposition is performed, for example, by the transfer in or onto the container of a predetermined quantity of chemical compound set to spread over a predetermined surface. Such predetermination allows for the comparison of results as evaporation is in part due to the size surface of the compound in contact with the ambient environment. The more parameters are set and predetermined, the more accurate the evaporation rate measurement is.
The transfer of the quantity of chemical compound can be performed using any known means to transfer liquids, preferably in small quantity, such as a pipette. Such a transfer can be performed manually or in an automatic manner.
The chemical compound considered can be in the form of a liquid or in the form of a solid diluted into a liquid. Preferably, such a chemical compound is pure. "Pure", in this context, is intended as meaning "overwhelmingly containing said chemical compound".
This step 105 of controlled deposition is preferably performed at a controlled temperature throughout the evaporation quantity measurement.

Evaporation rates are preferably measured in pseudo-equilibrium conditions: controlled temperature, air flow and rate, to basically mimic a closed thermodynamic system. Such an approach confirms that the evaporation rates can easily be related to a thermodynamic quantity such as vapor pressure.

The step 110 of airflow generation is performed, for example, using a pump or other airflow generation means. Preferably, the airflow is representative of the average airflow onto the skin of a person. In variants, several measurements 115 are made for a set number of airflow strengths to calculate 120 the impact of the airflow onto the evaporation rate of compounds.

The step 115 of measurement is performed, for example, using a microbalance chemical compound sensor downstream of the deposit of compound along the airflow. Such a sensor is configured to determine a quantity of compound sensed over time, which allows for the determination of the quantity of evaporated chemical compound evaporated.

Alternatively, the step 115 of measurement is performed by rising the remaining materials with a solvent, or by trapping in a cartridge the evaporated materials followed by quantification by gaseous phase chromatography.

The step 120 of evaporation rate calculation is performed, for example, by a computing means, such as a computer or server depending on the nature of the information architecture of the particular embodiment considered. The evaporation rate calculated corresponds to the variation of the amount of a chemical compound measured during a given time period divided by the length of said time period.

The evaporation rate can thus be a function of:

- temperature,
- surface of the container,
- quantity of compound deposited and
- air flow volume.

Preferably, the maximum evaporation rate is used. Said maximum evaporation rate is considered when at a 100% concentration of an ingredient. If an ingredient is diluted, the evaporation rate will be proportionally lowered.

The evaporation rate is constant all along the measurement time in the pseudo-equilibrium conditions (constant mass, temperature, constant pressure). The loss of mass via evaporation is so low that is do not affect the system, and the measurement is very close to the equilibrium conditions.

The step 125 of volatility calculation is performed, for example, by a computing means, such as a computer or server depending on the nature of the information architecture of the particular embodiment considered. In order to obtain such a volatility, a linear regression model linking evaporation rate to concentration is preferably obtained. Such a model can be achieved by measuring the evaporation rate and relative gas phase concentrations for several initial ingredient concentration in the liquid or solid phase at preset experimental conditions corresponding to equilibrium conditions. This allows for the building of a linear model linking evaporation rate to concentration at equilibrium.

Using the above-mentioned model, volatility is measured using a linear regression function that converts the pseudo-equilibrium evaporation rates (in standard conditions) into volatility.

In this embodiment, evaporation rate is measured, and then converted into a volatility using said linear regression function. Such a relationship between the evaporation rate at pseudo-equilibrium conditions and volatility at equilibrium is a discovery made by the inventors.

Volatility is preferably measured for a pure compound, which corresponds to the maximum evaporation rate of said compound at equilibrium.

The step 130 of storing is performed, for example, by a computerized database accessible by the computing means configured to perform the evaporation rate and volatility calculation computing. Such a database can be stored on a server, for example.

In more advanced embodiments, the method 100 further comprises a plurality of steps 105 of controlled deposition of a chemical compound at different temperature, the evaporation rate being calculated for each said temperature and stored during the step 130 of storing.

In particular embodiments, the method 100 further comprises:

- a step 135 of computing at least one gas phase concentration of a chemical compound for a given volatility of chemical compound,
- a step 140 of measurement of the psychophysical intensity of a chemical compound for at least one said gas phase concentration,
- a step 145 of modeling of a mathematical formula of psychophysical intensity as a function of gas phase concentration based on at least two of the measured gas phase concentration values and
- a step 150 of recording, in a database, the psychophysical intensity formula modeled parameters.

The gas phase concentration can vary from zero to the maximum concentration of the compound which is equal to the compound's volatility.

The step 135 of computing at least one gas phase concentration is performed, for example, by a computing means, such as a computer or server depending on the nature of the information architecture of the particular embodiment considered. During this step 135 of computing, as a function of the volatility which itself is a maximum concentration. Such values can be, for example, set to volatility, half of volatility or hundredth of volatility for example.

In variants, the step 135 of computing uses an indirect computing method, in which at least one concentration value is approximated from a quantity of compound deposited over a smelling strip. As such, the step 135 of computing can use a direct or indirect computing method.

It is this concentration that can then be presented to a user by inserting a quantity of evaporated chemical compound into an airflow thus allowing for the computing of the gas phase concentration of chemical compound. This gas phase concentration of chemical compound is considered as the ratio of the quantity of chemical compound to the volume of air.

In more advanced embodiments, the chemical compound is not provided as already evaporated but in liquid form and the airflow is configured to carry the evaporated content of the liquid chemical compound. In such an embodiment, setting the volume of the airflow allows for the use of the calculated corresponding evaporation rate to determine, for a given time, the amount of chemical compound evaporated.

The step 140 of measurement of the psychophysical intensity of a chemical compound if performed, for example, empirically by registering an input representative of perceived intensity by a panel of users. Such an input can be registered via a human-machine interface of any kind. Such input is stored into a registry. Preferably, this step 140 of measurement is performed at various given gaseous concentrations.

The step 145 of modeling is performed, for example, by a computing means, such as a computer or server depending on the nature of the information architecture of the particular embodiment considered. Such a modeling intends to perform a fit between a mathematical formula and the sample data representative of the mean perceived intensity by a panel of users. Such a mathematical formula can be a sigmoid curve for example in which parameters are set to match the perceived intensities.

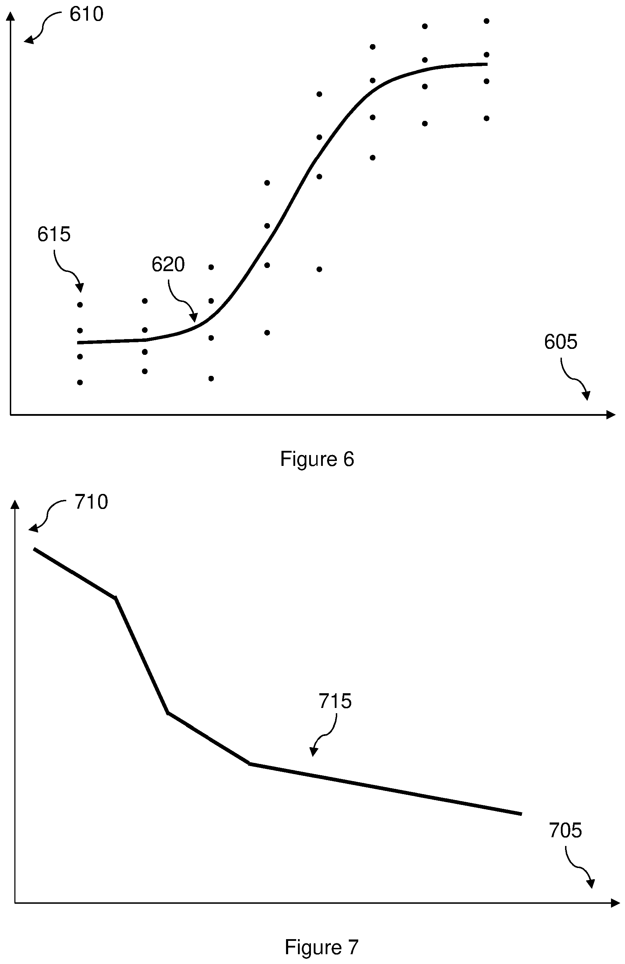

Such a sigmoid curve can be seen in figure 6 which shows:

- an axis representative of increasing gas phase concentration of a chemical compound modeled in logarithmic scale,
- an axis representative of increasing perceived psychophysical intensity,
- for given gas phase concentrations, a sample of perceived intensity by a pool of users and
- a sigmoid curve fitting the sample distribution for given gas phase concentrations.

Such a modeled curve is called a "dose-response curve".

Preferably, the cycle of the steps of computing a gas phase concentration 135, measurement 140 and modeling 145 are repeated for several gas phase concentrations of a given compound. For instance, a chemical compound can be tested using this methodology for gas phase concentrations ranging from zero to the compound volatility.

Preferably, at least two gas phase concentrations of a given compound are handled this way. Preferably, at least two gas phase concentrations of a given compound are handled this way. Preferably, at least eight gas phase concentrations of a given compound are handled this way. The more gas phase concentration evaluated, the more accurate the modeling.

The step of recording 150 is functionally analog to the step of storing 130.

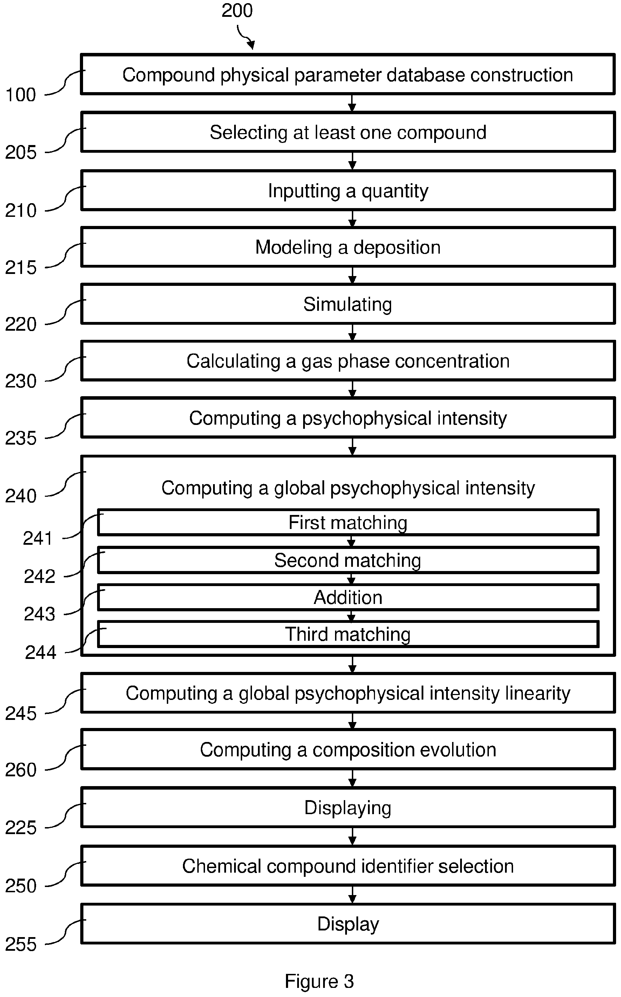

Figure 3 shows a particular succession of steps of a method which is the subject of this invention. This fragrance physical composition evolution prediction method 200 to provide predictive, real-time, fragrance performance metrics, comprises:

- a step 205 of selecting at least one chemical compound identifier in a computerized interface,
- a step 210 of inputting, for each selected chemical compound, a quantity of said chemical compound,
- a step 215 of modeling a deposition of the quantity of each selected chemical compound on a virtual surface,
- a step 220 of simulating, by a computing system, for at least one modeled deposited chemical compound, the stripped quantity of said chemical compound in an airflow for at least two different times as a function of:

    - the quantity of each said chemical compound,
    - a first value representative of a virtual surface size of the deposited chemical compound,
    - a second value representative of a virtual airflow directed at the deposited chemical compound configured to virtually strip the chemical compound from the surface,
    - a third value representative of an activity coefficient of each said chemical compound and
    - an evaporation rate or volatility associated to said chemical compound stored in a database constructed according to the database construction method of figure 2 and

- a step 225 of displaying, for each chemical compound, of an indicator representative of the computed evaporated mass of said compound over time.

The step 205 of selecting at least one chemical compound identifier is performed by any human-machine interface allowing for the selection of at least one such identifier. In one particular embodiment, said human-machine interface is a mouse and/or keyboard allowing for the selection, in an interface, of at least one identifier. Such identifier can be a compound name, a compound logo or icon or any reference in a compound classification system.

The step 210 of inputting can be either automatic or manual. In the event where the step is performed manually, by an operator, this step 210 of inputting is performed by any human-machine interface allowing for the input of said quantity. Such a human-machine interface can be a mouse and/or keyboard allowing for the input, in an interface, of said quantity.

It should be noted that the term quantity designates either an absolute value, in grams or mols or liters, or a relative value, in parts of the overall composition. For example, a composition comprising 15 parts of compound A and 10 of compound B allows, after determination of the overall volume of the composition, to determine the quantity of compounds A and B. Such parts can be expressed in volume, molar quantities or mass for example.

The step 215 of modeling is performed, for example, by a computing means, such as a computer or server depending on the nature of the information architecture of the particular embodiment considered. In particular embodiments, during this step, parameters are set, these parameters corresponding to the intended use of the composition. For example, these parameters can correspond to an intended airflow over the composition or to an intended surface of dispersion of said compound. In variants, more complex parameters could be set, such as the distance of dispersion of the composition, from which a surface of deposition can be inferred. Such parameters can be automatically or manually set. In automatic variants, an operator can select a parameter setting profile which automatically sets at least one parameter value.

The step 220 of simulating is performed, for example, by a computing means, such as a computer or server depending on the nature of the information architecture of the particular embodiment considered.

During this step 220 of simulating, it is key to understand the difference between traditional diffusion evaluation methods and stripping evaluation. Diffusion corresponds to the sole consideration of evaporation as a means for transporting a compound from a liquid bulk to a gaseous volume. Such models are extremely imperfect in their modeling of compositions that are spread over a surface, such as a perfume over skin.

On skin, in a real fragrance application there exists only a very thin layer of fragrance, that is spread depending on the way the fragrance is applied, the quantity that is applied, the viscosity of the fragrance, the dilution in ethanol and other such parameters. What happens is that this spread defines the surface of the fragrance to evaporate, and that surface is a dominant factor that defines fragrance release: the bigger the surface, the faster a fragrance release will be.

This is why it makes more physical sense to evaluate a fragrance stripping, as is defined in the Chemical engineering textbook: "Stripping or desorption is the transfer of gas, dissolved in a liquid, into a gas stream". It is this phenomenon that is simulated here.

Another way of understanding this phenomenon is to consider that this thin layer of volatile compounds is actually a gas dissolved in a liquid, as volatiles exist as both gas and liquid at the conditions of wearing a fragrance (obviously, as a fragrance is present in the air, at a comparable composition of ingredients as on skin).

The modeled stripping process corresponds to a fragrance release from skin that considers:

- the perfume spread - the surface of the spread perfume, which affects strongly the fragrance release,
- the air convection which is a dominant force that is responsible to for the mass transfer liquid/gas,
- compound volatility obtained by a conversion function from empirically measured evaporation rates and not theoretical vapor pressures or equilibrium vapor pressure that is defined as the pressure exerted by a vapor in thermodynamic equilibrium with its condensed phases (solid or liquid) at a given temperature in a closed system. Instead, this model uses evaporation rates, which is the speed by which an ingredient evaporates expressed as mass/time unit,
- preferably, the equilibrium conditions are considered at small time steps, and the fragrance composition on skin is predicted in time steps and
- the gas phase concentration is modeled by using the air convection - not the diffusion based upon a determined airflow.

Such a model is new, distinctly different from the diffusion models using Stefan tubes to be modelled, as it is describing a different phenomenon - stripping of thin layer of gas dissolved in a liquid, which reflects the reality of wearing a fragrance, over a period of 6-8 hours.

Such a model uses, for example, the following formula to evaluate the stripped mass of a compound at a given time interval:

$$\frac{dm_i}{dt} = \frac{D_i}{e} . A . x_i . \gamma_i . Vol_i$$

In which:

- $D_i$ is the diffusion coefficient in air of the compound (in $m^2s^{-1}$),
- e is the stagnant layer thickness, representing the second value representative of a virtual airflow notably,
- $A$ (t) is the evaporation surface (in $m^2$), or a value representative of a virtual surface size of the deposited chemical compound,
- $x_i$(t) is the liquid mole fraction of the compound,
- $\gamma_i$(t) is the activity coefficient of the compound, which can be set at a value of one for example and

- *Vol_i* is the volatility of the compound (in micrograms/m³ air).

The validation of such models also use-a value representative of a surface characteristic of the virtual surface upon which the chemical compound is deposited and stripped from over time, said computing parameter being configured to provide human skin like chemical compound interaction properties.

These values can be set to common or separate empirical constant for simplicity.

In such a simplified model, the equation can be set as:

$$\frac{dm_i}{dt} = K \cdot \frac{m_i(t)}{sum\ (t)} \cdot Vol_i$$

In which:

- *K* is a constant of evaporation representative of specific experimental conditions mimicking fragrance evaporation from skin, K is an empirical value obtained by fitting perfume evaporation experiments in standard condition with the model,
- *m_i(t)* is the mass of compound i at time t,
- *sum* (t) is the total mass of all compounds at time t and
- *Vol_i* is the volatility of the compound (in micrograms/m³ air).

Calculations of the step 220 of simulating can be performed after compounds have been selected and quantities set or prior, in which case calculation results are stored and addressed after compounds have been selected and quantities set.

The step 225 of displaying is performed, for example, by a screen configured to display a user interface in which an operator may see the results of the simulation.

Figure 7 represents, a sample curve representative of the remaining mass of a chemical compound at different time steps in which:

- the horizontal axis 705 represents time, measured in time steps,
- the vertical axis 710 represents the remaining quantity of compound and
- a curve 715 or curve approximation or extrapolated curve or interpolated curve is shown.

In particular embodiments, the method 200 of figure 3 further comprises a step 230 of computing a gas phase concentration of the virtually stripped chemical compound as a function of the stripped quantity computed, the step 225 of displaying being configured to display the gas phase concentration computed.

The step 230 of computing a gas phase concentration is performed, for example, by a computing means, such as a computer or server depending on the nature of the information architecture of the particular embodiment considered.

In such a step 230 of computing, a fixed air volume in which the stripping is happening is preferably set. The more compound is stripped off the surface, the more the gas phase concentration increases. The gas phase concentration is calculated by dividing the total stripped mass in a given time period by the air volume stripping the mass over that time period. More complex embodiments provide a dynamic air volume increasing with time, the stripped mass of each time interval being divided by the maximum air volume.

It should be noted that chemical compounds in a composition are competing for the capacity to evaporate and thus the evolution of the compound formulation and relative quantity ratio between the compounds alters the concentration of each compound in air which, in turn, alters the perceived scent of the composition.

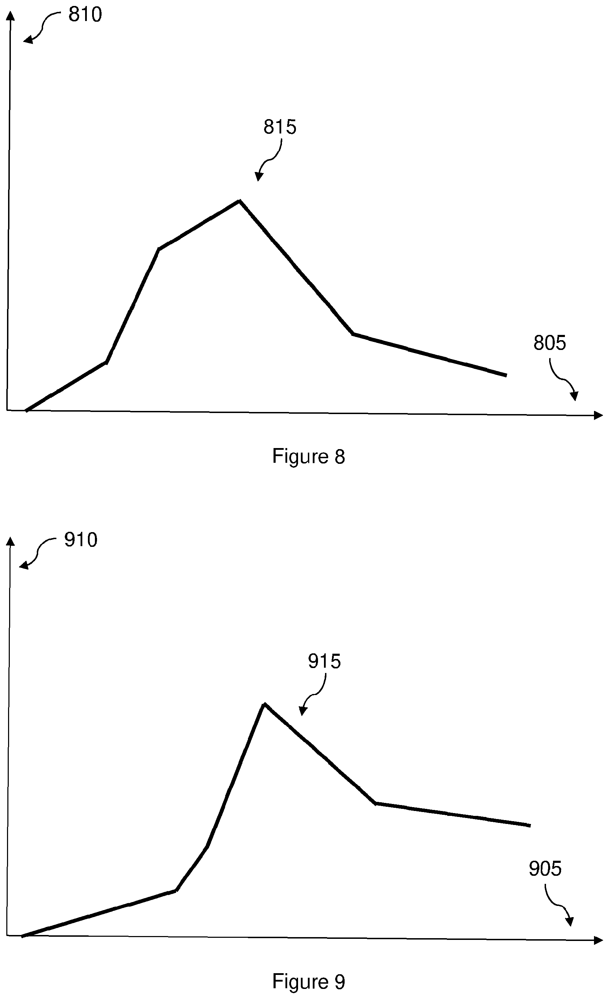

The result of such a step 230 of computing is shown in figure 8, which shows:

- the horizontal axis 805 represents time, measured in time steps,
- the vertical axis 810 represents the concentration of compound and
- a curve 815 or curve approximation or extrapolated curve or interpolated curve is shown.

Figure 8 shows that the concentration of a compound in the airspace can evolve through time in a given composition formula in which, for instance, the studied compound might be stripped in priority in the first moments after composition dispersion. After a given time, this first compound can be stripped in a secondary manner as opposed to a second compound. Therefore, compound interaction is very impactful on the performance of a composition in terms of relative compound concentrations in the headspace over time.

In particular embodiments, the method 200 of figure 3 further comprises a step 235 of computing a psychophysical intensity of each selected chemical compound as a function of the gas phase concentration computed, the step 225 of displaying being configured to display the psychophysical intensity computed over time.

The step 235 of computing a psychophysical intensity is performed, for example, by a computing means, such as a computer or server depending on the nature of the information architecture of the particular embodiment considered.

Such a step 235 of computing can be performed by matching the gas phase concentration of the compound with the corresponding dose-response curve values.

The result of such a step 235 of computing is shown in figure 9, which shows:

- the horizontal axis 905 represents time, measured in time steps,
- the vertical axis 910 represents the perceived intensity of the chemical compound at said time step and
- a curve 915 or curve approximation or extrapolated curve or interpolated curve is shown.

In particular embodiments, at least two chemical compounds are selected, the method 200 shown in figure 3 further comprising a step 240 of computing a global psychophysical intensity of the concentration of each chemical compound selected, the step 225 of displaying being configured to display the global psychophysical intensity computed over time.

It should be understood at this stage that the psychophysical intensity of a compound at a given gas phase concentration is better expressed when the psychophysical intensity is considered logarithmically.

To evaluate the global intensity of a composition constituted of several fragrances, the step 240 of computing a global psychophysical intensity may further comprise:

- a first step 241 of matching a value for concentration for each compound against the corresponding dose-response curve to provide a perceived intensity value for that compound,
- a second step 242 of matching each said perceived intensity value against a dummy dose-response curve to provide an artificial compound concentration value,
- a step 243 of addition of each artificial compound concentration value to form a virtual concentration value,
- a third step 244 of matching the virtual concentration value against the dummy dose-response curve to provide a total perceived intensity value for the composition.

This is vastly different from prior art that uses Steven's power law, which is an oversimplification stating that the intensity of the mixture corresponds to the intensity of the most intense ingredient. Such a method yields results that are verified empirically.

Particular embodiments further comprise a step 240 of computing a global odor intensity comprising:

- a first step of matching a value for concentration for each compound against the corresponding dose-odor descriptor intensity curve to provide a perceived intensity of an odor value for that compound,
- a second step of matching each said perceived intensity value against a dummy dose-odor descriptor intensity curve to provide an artificial compound concentration value,
- a step of addition of each artificial compound concentration value to form a virtual concentration value and

- a third step of matching the virtual concentration value against the dummy dose-odor descriptor intensity curve to provide a total perceived odor descriptor intensity value for the composition.

It should be understood that such embodiments require the prior modeling of the mathematical formula of the odor descriptor intensity in relation to the concentration. Such a formula can be garnered from empirical measurement of psychophysical intensity for a given odor at different compound concentration levels.

In further embodiments, if a compound is associated to a plurality of odors, the concentration of said compound may be divided by two or according to a particular weighing rule prior to the first step of matching.

The step 240 of computing a computing a global psychophysical intensity is performed, for example, by a computing means, such as a computer or server depending on the nature of the information architecture of the particular embodiment considered.

In particular embodiments, at least two chemical compounds are selected, the method 200 shown in figure 3 further comprising a step 245 of computing a psychophysical intensity linearity of the composition of said at least two compounds based on the computed psychophysical intensity of each selected chemical compound over time, the step 225 of displaying being configured to display the psychophysical intensity linearity of the composition of said at least two compounds.

Linearity can be understood as a measure of the uniformity of the relative psychophysical intensities in a composition over time. If compound A is perceived as twice as intensely as compound B for most of the time intervals, then the composition is more linear than if compound B becomes more perceivable than compound A after a given time interval.

The step 245 of computing a psychophysical intensity linearity is performed, for example, by a computing means, such as a computer or server depending on the nature of the information architecture of the particular embodiment considered.

In particular embodiments, the method 200 shown in figure 3 further comprises a step 250 of chemical compound identifier selection, said chemical compound identifier being selected if the psychophysical intensity at a given time is below a determined value and a step 255 of display of said chemical compound identifier.

The step 250 of selecting is performed, for example, by a computing means, such as a computer or server depending on the nature of the information architecture of the particular embodiment considered. This step 250 of selection is preferably performed automatically.

During this step 250 of selecting, if a computed psychophysical intensity drops below a specified threshold, statically or dynamically set, the corresponding compound is selected. In dynamically set thresholds, the threshold varies with the highest recorded psychophysical intensity for a given time interval, for example.

This allows for the prediction of compounds that are failing in terms of perceived intensity at a given time from initial deposit.

The step 255 of display is analogous to the step 225 of displaying, albeit in another interface or element of interface for example.

In particular embodiments, at least two chemical compounds are selected to form a composition, the method 200 shown in figure 3 further comprising a step 260 of computing of the composition evolution over time as a function of the stripped quantity calculated over time.

The step 260 of computing of the composition evolution over time is performed, for example, by a computing means, such as a computer or server depending on the nature of the information architecture of the particular embodiment considered.

In such a step 260 of computation, the stripped quantity of each compound is simulated, allowing for the determination of the remaining quantity of each compound in liquid state and thus the composition of the liquid state.

The composition evolution can be measured, for example, in relative quantity of compounds not yet in suspension in the volume of air.

In particular embodiments, the method 200 of figure 3 further comprises a liquid chemical compound physical parameter database construction step 100 comprising:

- a step 105 of controlled deposition of a chemical compound in an inert container,
- a step 110 of airflow generation, the airflow being directed in the direction of the deposited chemical compound,
- a step 115 of measurement of a quantity of evaporated chemical compound at different measurement times,
- a step 120 of evaporation rate calculation depending on the measured evaporated chemical compound quantities measured,
- a step 125 of volatility calculation depending on the evaporation rate calculated and
- a step 130 of storing, in a database, the calculated evaporation rate and the volatility calculated.

Such steps are disclosed in regard to figure 2.

It should be understood that such a process 200 could be used in such manner:

- a user of a perfume design interface logs into the platform via a computerized interface,
- the user creates a new perfume by creating a composition containing at least one compound,
- the user specifies intended compound quantities, either relatively or in absolute terms,
- a computing architecture then computes the evaporated quantity of each compound for a determined number of time steps,
- the interface shows one or more graphs showing the evolution of the composition in terms of compound composition, or the evolution of gas phase concentration or perceived intensity for a compound or the composition as a whole.

The current invention allows augmented fragrance creation by predicting fragrance creation outcome, fragrance lastingness and evolution of formula, smell and intensity over time for example.

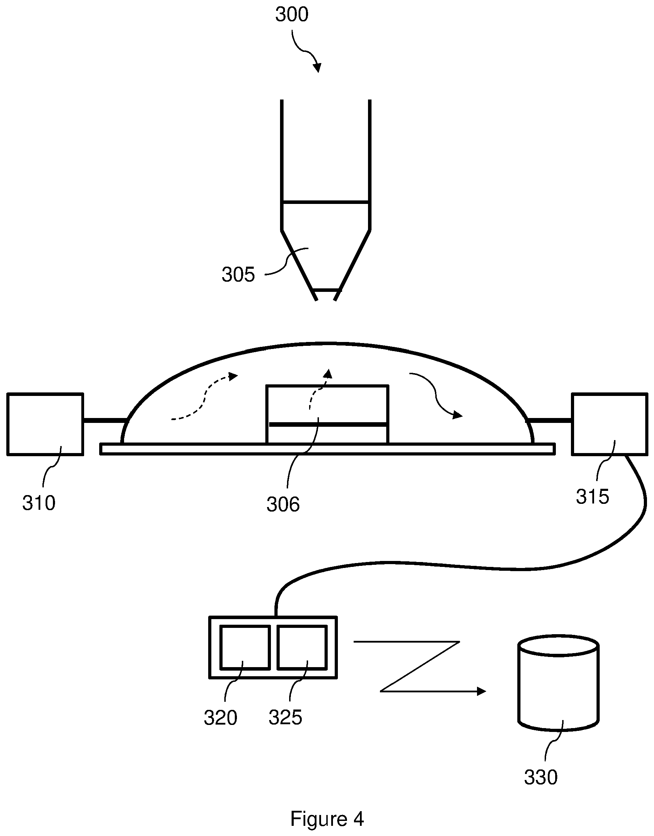

Figure 4 shows, schematically and not to scale, a particular embodiment of a system 300 object of the present invention. This liquid chemical compound physical parameter database construction system 300, comprises:

- a means 305 of controlled deposition of a chemical compound in an inert container 306,
- a means 310 of airflow generation, the airflow being directed in the direction of the deposited chemical compound,
- a means 315 of measurement of a quantity of evaporated chemical compound at different measurement times,
- a means 320 of evaporation rate calculation depending on the measured evaporated chemical compound quantities measured,
- a means 325 of volatility calculation depending on the evaporation rate calculated and
- a means 330 of storing, in a database, the calculated evaporation rate and the volatility calculated.

The means 305 of controlled deposition corresponds to variants disclosed relative to the step 105 of controlled deposition shown in figure 2. Such a means 305 is, for example, a manual or automated pipette.

The means 310 of airflow generation corresponds to variants disclosed relative to the step 110 of airflow generation shown in figure 2. Such a means 310 is, for example, a pump.

The means 315 of measurement corresponds to variants disclosed relative to the step 115 of measurement shown in figure 2. Such a means 315 is, for example, a compound presence and quantity sensor.

The means 320 of evaporation rate calculation corresponds to variants disclosed relative to the step 120 of evaporation rate calculation shown in figure 2. Such a means 320 is, for example, a computer or server.

The means 325 of volatility calculation corresponds to variants disclosed relative to the step 125 of volatility calculation shown in figure 2. Such a means 325 is, for example, a computer or server.

The means 330 of storing corresponds to variants disclosed relative to the step 330 of storing shown in figure 2. Such a means 330 is, for example, a database accessible on an information network.

Figure 4 shows, schematically and not to scale, a particular embodiment of a system 400 object of the present invention. This fragrance physical parameter evolution prediction system 400 to provide predictive, real-time, fragrance performance metrics, comprises:

- a means 405 of selecting at least one chemical compound identifier in a computerized interface,
- a means 410 of inputting, for each selected chemical compound, a quantity of said chemical compound,
- a means 415 of modeling a deposition of the quantity of each selected chemical compound on a virtual surface,
- a means 420 of simulating, by a computing system, for at least one modeled deposited chemical compound, the stripped quantity of said chemical compound in an airflow for at least two different times as a function of:

  - the quantity of each said chemical compound,
  - a first value representative of a virtual surface size of the deposited chemical compound,
  - a second value representative of a virtual airflow directed at the deposited chemical compound configured to virtually strip the chemical compound from the surface,
  - a third value representative of an activity coefficient of each said chemical compound,
  - an evaporation rate associated to said chemical compound stored in a database constructed according to the database construction method of figure 2,
  - a fourth value representative of a surface characteristic of the virtual surface upon which the chemical compound is deposited and stripped from over time, said computing parameter being configured to provide human skin like chemical compound interaction properties and

- a means 425 of displaying, for each chemical compound, of an indicator representative of the computed evaporated mass of said compound over time.

The means 405 of selecting corresponds to variants disclosed relative to the step 205 of selecting shown in figure 3. Such a means 405 is, for example, a keyboard and/or a mouse allowing for the control of a computerized interface.

The means 410 of inputting corresponds to variants disclosed relative to the step 210 of inputting shown in figure 3. Such a means 410 is, for example, a keyboard and/or a mouse allowing for the control of a computerized interface.

The means 415 of modeling corresponds to variants disclosed relative to the step 215 of modeling shown in figure 3. Such a means 410 is, for example, a computer or server.

The means 420 of modeling corresponds to variants disclosed relative to the step 220 of modeling shown in figure 3. Such a means 420 is, for example, a computer or server.

The means 425 of displaying corresponds to variants disclosed relative to the step 225 of displaying shown in figure 3. Such a means 425 is, for example, a computer screen.

**Claims**

1. A volatile liquid chemical compound physical parameter database construction method (100), **characterized in that** it comprises:

   - a step (105) of controlled deposition of a chemical compound in an inert container, a predetermined quantity of the chemical compound spreading over a predetermined surface of the container,
   - a step (110) of airflow generation, the airflow being directed in the direction of the deposited chemical compound,
   - a step (115) of measurement of a quantity of evaporated chemical compound at different measurement times,
   - a step of measurement of relative gas phase concentrations for several initial ingredient concentration in the liquid chemical compound at preset experimental pseudo-equilibrium conditions corresponding to equilibrium conditions,
   - a step (120) of evaporation rate calculation depending on the measured evaporated chemical compound quantities measured at pseudo-equilibrium conditions,
   - a step (125) of volatility calculation depending on the evaporation rate calculated, a first linear regression function linking evaporation rate to concentration at equilibrium conditions and a second linear regression function that converts pseudo-equilibrium evaporation rates into volatility and
   - a step (130) of storing, in a database, the calculated evaporation rate and the volatility calculated.

2. A construction method (100) according to claim 1, which further comprises:

   - a step (135) of computing at least one gas phase concentration of the chemical compound for a given volatility of chemical compound,
   - a step (140) of measurement of the psychophysical intensity of the chemical compound for at least one said gas phase concentration,
   - a step (145) of modeling of a mathematical formula of psychophysical intensity of the chemical compound as a function of gas phase concentration based on at least two of the measured gas phase concentration values named "dose-response curve" and
- a step (150) of recording, in a database, the psychophysical intensity formula modeled parameters of the chemical compound.

3. A construction method (100) according to claim 1 or 2, which comprises a plurality of steps (105) of controlled deposition of a chemical compound at different temperature, the evaporation rate being calculated for each said temperature and stored during the step of storing.

4. A fragrance physical composition evolution prediction method (200) to provide predictive, real-time, fragrance performance metrics, **characterized in that** it comprises:

- a step (205) of selecting at least one chemical compound identifier in a computerized interface,
- a step (210) of inputting, for each selected chemical compound, a quantity of said chemical compound,
- a step (215) of modeling a deposition of the quantity of each selected chemical compound on a virtual surface by setting at least one parameter of the model,
- a step (220) of simulating, by a computing system, for each modeled deposited chemical compound, the stripped quantity of said chemical compound in an airflow for at least two different times as a function of:

- the quantity of each said chemical compound,
- a first value representative of a virtual surface size of the deposited chemical compound,
- a second value representative of a virtual airflow directed at the deposited chemical compound configured to virtually strip the chemical compound from the surface,
- a third value representative of an activity coefficient of each said chemical compound and
- a volatility associated to said chemical compound stored in a database constructed according to the database construction method of any one of claims 1 to 3 and

- a step (225) of displaying, for each chemical compound, of an indicator representative of the computed evaporated mass of said compound over time.

5. A prediction method (200) according to claim 4, further comprising a step (230) of computing a gas phase concentration of the virtually stripped chemical compound as a function of the stripped quantity computed, the step (225) of displaying being configured to display the gas phase concentration computed.

6. A prediction method (200) according to claim 5 when claim 4 depends on claim 2, further comprising a step (235) of computing a psychophysical intensity of each selected chemical compound as a function of the gas phase concentration computed, the step (225) of displaying being configured to display the psychophysical intensity computed over time.

7. A prediction method (200) according to claim 6, in which at least two chemical compounds are selected, the method further comprising a step (240) of computing a global psychophysical intensity comprising:

- a first step (241) of matching a value for concentration for each compound against the corresponding dose-response curve to provide a psychophysical intensity value for that compound,
- a second step (242) of matching each said psychophysical intensity value against a dummy dose-response curve to provide an artificial compound concentration value,
- a step (243) of addition of each artificial compound concentration value to form a virtual concentration value and
- a third step (244) of matching the virtual concentration value against the dummy dose-response curve to provide a global psychophysical intensity value for the composition.

the step of displaying being configured to display the global psychophysical intensity computed over time.

8. A prediction method (200) according to either claim 6 or 7, in which at least two chemical compounds are selected, the method further comprising a step (245) of computing a psychophysical intensity linearity of the composition of said at least two compounds based on the computed psychophysical intensity of each selected chemical compound over time, the step (225) of displaying being configured to display the psychophysical intensity linearity of the composition of said at least two compounds.

9. A prediction method (200) according to any one of claims 6 to 8, which further comprises a step (250) of chemical compound identifier selection, said chemical compound identifier being selected if the psychophysical intensity at a given time is below a determined value and a step (255) of display of said chemical compound identifier.

10. A prediction method (200) according to any one of claims 4 to 9, in which at least two chemical compounds are selected to form a composition, the method further comprising a step (260) of computing of the composition evolution over time as a function of the stripped quantity calculated over time.

11. A prediction method (200) according to any one of claims 4 to 9, which further comprises a step of construction of the liquid chemical compound physical parameter database storing the volatility associated to said chemical compound by carrying out the method of claims 1 to 3.

12. A liquid chemical compound physical parameter database construction system (300), **characterized in that** it comprises:

- a means (305) of controlled deposition of a chemical compound in an inert container (306),
- a means (310) of airflow generation, the airflow being directed in the direction of the deposited chemical compound,
- a means (315) of measurement of a quantity of evaporated chemical compound at different measurement times,
- a means of measurement of relative gas phase concentrations for several initial ingredient concentration in the liquid chemical compound at preset experimental pseudo-equilibrium conditions corresponding to equilibrium conditions,
- a means (320) of evaporation rate calculation depending on the measured evaporated chemical compound quantities measured at pseudo-equilibrium conditions,
- a means (325) of volatility calculation depending on the evaporation rate calculated, a first linear regression function linking evaporation rate to concentration at equilibrium conditions and a second linear regression function that converts pseudo-equilibrium evaporation rates into volatility and
- a means (330) of storing, in a database, the calculated evaporation rate and the volatility calculated.

13. A fragrance physical parameter evolution prediction system (400) to provide predictive, real-time, fragrance performance metrics, **characterized in that** it comprises:

- a means (405) of selecting at least one chemical compound identifier in a computerized interface,
- a means (410) of inputting, for each selected chemical compound, a quantity of said chemical compound,
- a means (415) of modeling a deposition of the quantity of each selected chemical compound on a virtual surface by setting at least one parameter of the model,
- a means (420) of simulating, by a computing system, for each modeled deposited chemical compound, the stripped quantity of said chemical compound in an airflow for at least two different times as a function of:

- the quantity of each said chemical compound,
- a first value representative of a virtual surface size of the deposited chemical compound,
- a second value representative of a virtual airflow directed at the deposited chemical compound configured to virtually strip the chemical compound from the surface,
- a third value representative of an activity coefficient of each said chemical compound and
- a volatility associated to said chemical compound stored in a database constructed according to the database construction method of any one of claims 1 to 3 and

- a means (425) of displaying, for each chemical compound, of an indicator representative of the computed evaporated mass of said compound over time.

**Patentansprüche**

1. Datenbankaufbauverfahren für eine Datenbank der physikalischen Parameter von flüchtigen flüssigen chemischen Verbindungen (100), **dadurch gekennzeichnet, dass** es umfasst:

- einen Schritt (105) zur kontrollierten Ablagerung einer chemischen Verbindung in einem inerten Behälter, wobei sich eine vorbestimmte Menge der chemischen Verbindung über eine vorbestimmte Oberfläche des Behälters verteilt,
- einen Schritt (110) zur Erzeugung eines Luftstroms, wobei der Luftstrom in der Richtung der abgelagerten chemischen Verbindung gerichtet ist,
- einen Schritt (115) zur Messung einer Menge einer verdampften chemischen Verbindung zu verschiedenen Messzeitpunkten,
- einen Schritt zur Messung relativer Gasphasenkonzentrationen für mehrere anfängliche Inhaltsstoffkonzentrationen in der flüssigen chemischen Verbindung unter voreingestellten experimentellen Pseudogleichgewichtsbedingungen, welche Gleichgewichtsbedingungen entsprechen,

- einen Schritt (120) zur Berechnung einer Verdampfungsrate in Abhängigkeit von den gemessenen Mengen verdampfter chemischer Verbindungen, welche unter Pseudogleichgewichtsbedingungen gemessen wurden,
- einen Schritt (125) zur Berechnung einer Flüchtigkeit in Abhängigkeit von der berechneten Verdampfungsrate, einer ersten linearen Regressionsfunktion, welche Verdampfungsrate mit Konzentration unter Gleichgewichtsbedingungen verknüpft, und einer zweiten linearen Regressionsfunktion, welche Pseudogleichgewichtsverdampfungsraten in Flüchtigkeit umwandelt, und
- einen Schritt (130) zum Speichern der berechneten Verdunstungsrate und der berechneten Flüchtigkeit in einer Datenbank.

2. Aufbauverfahren (100) nach Anspruch 1, weiter umfassend:

- einen Schritt (135) zum Berechnen mindestens einer Gasphasenkonzentration der chemischen Verbindung für eine gegebene Flüchtigkeit einer chemischen Verbindung,
- einen Schritt (140) zur Messung der psychophysischen Intensität der chemischen Verbindung für mindestens eine der Gasphasenkonzentrationen,
- einen Schritt (145) zum Modellieren einer mathematischen Formel einer psychophysischen Intensität der chemischen Verbindung als eine Funktion von Gasphasenkonzentration basierend auf mindestens zwei der gemessenen Gasphasenkonzentrationswerte, "Dosis-Wirkungs-Kurve" genannt, und
- einen Schritt (150) zum Aufzeichnen der durch die psychophysische Intensitätsformel modellierten Parameter der chemischen Verbindung in einer Datenbank.

3. Aufbauverfahren (100) nach Anspruch 1 oder 2, welches eine Vielzahl von Schritten (105) zur kontrollierten Ablagerung einer chemischen Verbindung bei verschiedenen Temperaturen umfasst, wobei die Verdampfungsrate für jede Temperatur berechnet und während des Speicherschritts gespeichert wird.

4. Vorhersageverfahren für die Entwicklung der physikalischen Zusammensetzung (200) eines Duftstoffs, um in Echtzeit prädiktive Kennzahlen zur Duftleistung bereitzustellen, **dadurch gekennzeichnet, dass** es umfasst:

- einen Schritt (205) zum Auswählen mindestens einer Kennung einer chemischen Verbindung in einer computerisierten Schnittstelle,
- einen Schritt (210) zum Eingeben einer Menge der chemischen Verbindung für jede ausgewählte chemische Verbindung,
- einen Schritt (215) zum Modellieren einer Ablagerung der Menge jeder ausgewählten chemischen Verbindung auf einer virtuellen Oberfläche durch Einstellen mindestens eines Parameters des Modells,
- einen Schritt (220) zum Simulieren der abgestreiften Menge der chemischen Verbindung in einem Luftstrom für jede modellierte abgelagerte chemische Verbindung durch ein Computersystem für mindestens zwei verschiedene Zeitpunkte als eine Funktion von:

    - der Menge jeder der chemischen Verbindungen,
    - einem ersten Wert, welcher eine virtuelle Oberflächengröße der abgelagerten chemischen Verbindung darstellt,
    - einem zweiten Wert, der einen virtuellen Luftstrom darstellt, welcher auf die abgelagerte chemische Verbindung gerichtet ist und dazu konfiguriert ist, die chemische Verbindung virtuell von der Oberfläche abzustreifen,
    - einem dritten Wert, welcher einen Aktivitätskoeffizienten jeder der chemischen Verbindungen darstellt, und
    - einer mit der chemischen Verbindung verbundenen Flüchtigkeit, welche in einer Datenbank gespeichert ist, welche gemäß dem Datenbankaufbauverfahren nach einem der Ansprüche 1 bis 3 erstellt wurde, und

- einen Schritt (225) zum Anzeigen eines Indikators für jede chemische Verbindung, welcher die berechnete verdampfte Masse der Verbindung im Laufe der Zeit darstellt.

5. Vorhersageverfahren (200) nach Anspruch 4, welches weiter einen Schritt (230) zum Berechnen einer Gasphasenkonzentration der virtuell abgestreiften chemischen Verbindung als eine Funktion der berechneten abgestreiften Menge umfasst, wobei der Schritt (225) zum Anzeigen dazu konfiguriert ist, die berechnete Gasphasenkonzentration anzuzeigen.

6. Vorhersageverfahren (200) nach Anspruch 5, wenn Anspruch 4 von Anspruch 2 abhängt, welches weiter einen Schritt (235) zum Berechnen einer psychophysischen Intensität jeder ausgewählten chemischen Verbindung als eine Funktion der berechneten Gasphasenkonzentration umfasst, wobei der Schritt (225) zum Anzeigen dazu konfiguriert ist, die im Laufe der Zeit berechnete psychophysische Intensität anzuzeigen.

7. Vorhersageverfahren (200) nach Anspruch 6, bei welchen mindestens zwei chemische Verbindungen ausgewählt werden, wobei das Verfahren weiter einen Schritt (240) zum Berechnen einer globalen psychophysischen Intensität umfasst, welcher umfasst:

- einen ersten Schritt (241) zum Übereinstimmen eines Konzentrationswerts für jede Verbindung mit der entsprechenden Dosis-Wirkungs-Kurve, um einen psychophysischen Intensitätswert für diese Verbindung zu erhalten,
- einen zweiten Schritt (242) zum Übereinstimmen jeder der psychophysischen Intensitätswerte mit einer Dummy-Dosis-Wirkungs-Kurve, um einen künstlichen Konzentrationswert der Verbindung bereitzustellen,
- einen Schritt (243) zum Addieren jedes Konzentrationswerts einer künstlichen Verbindung, um einen virtuellen Konzentrationswert zu bilden, und
- einen dritten Schritt (244) zum Übereinstimmen des virtuellen Konzentrationswerts mit der Dummy-Dosis-Wirkungs-Kurve, um einen globalen psychophysischen Intensitätswert für die Zusammensetzung bereitzustellen.

wobei der Schritt zum Anzeigen dazu konfiguriert ist, die im Laufe der Zeit berechnete globale psychophysische Intensität anzuzeigen.

8. Vorhersageverfahren (200) nach Anspruch 6 oder 7, in welchem mindestens zwei chemische Verbindungen ausgewählt werden, wobei das Verfahren weiter einen Schritt (245) zum Berechnen einer psychophysischen Intensitätslinearität der Zusammensetzung der mindestens zwei Verbindungen basierend auf der berechneten psychophysischen Intensität jeder ausgewählten chemischen Verbindung im Laufe der Zeit umfasst, wobei der Schritt (225) zum Anzeigen dazu konfiguriert ist, die psychophysische Intensitätslinearität der Zusammensetzung der mindestens zwei Verbindungen anzuzeigen.

9. Vorhersageverfahren (200) nach einem der Ansprüche 6 bis 8, welches weiter einen Schritt (250) zur Auswahl einer Kennung für eine chemische Verbindung, wobei die Kennung für die chemische Verbindung ausgewählt wird, wenn die psychophysische Intensität zu einem gegebenen Zeitpunkt unter einem bestimmten Wert liegt, und einen Schritt (255) zum Anzeigen der Kennung für die chemische Verbindung umfasst.

10. Vorhersageverfahren (200) nach einem der Ansprüche 4 bis 9, in welchem mindestens zwei chemische Verbindungen ausgewählt werden, um eine Zusammensetzung zu bilden, wobei das Verfahren weiter einen Schritt (260) zum Berechnen der Entwicklung der Zusammensetzung im Laufe der Zeit als eine Funktion der im Laufe der Zeit berechneten abgestreiften Menge umfasst.

11. Vorhersageverfahren (200) nach einem der Ansprüche 4 bis 9, welches weiter einen Schritt zum Aufbauen einer Datenbank der physikalischen Parameter von flüssigen chemischen Verbindungen umfasst, in welcher die mit der chemischen Verbindung verbundene Flüchtigkeit gespeichert ist, indem das Verfahren nach Ansprüchen 1 bis 3 ausgeführt wird.

12. System zum Aufbau einer Datenbank der physikalischen Parameter von flüssigen chemischen Verbindungen (300), **dadurch gekennzeichnet, dass** es umfasst:

- ein Mittel (305) zur kontrollierten Ablagerung einer chemischen Verbindung in einem inerten Behälter (306),
- ein Mittel (310) zur Erzeugung eines Luftstroms, wobei der Luftstrom in der Richtung der abgelagerten chemischen Verbindung gerichtet ist,
- ein Mittel (315) zur Messung einer Menge einer verdampften chemischen Verbindung zu verschiedenen Messzeitpunkten,
- ein Mittel zur Messung relativer Gasphasenkonzentrationen für mehrere anfängliche Inhaltsstoffkonzentrationen in der flüssigen chemischen Verbindung unter voreingestellten experimentellen Pseudogleichgewichtsbedingungen, welche Gleichgewichtsbedingungen entsprechen,
- ein Mittel (320) zur Berechnung einer Verdampfungsrate in Abhängigkeit von den gemessenen Mengen verdampfter chemischer Verbindungen, welche unter Pseudogleichgewichtsbedingungen gemessen wurden,
- ein Mittel (325) zur Berechnung einer Flüchtigkeit in Abhängigkeit von der berechneten Verdampfungsrate, einer ersten linearen Regressionsfunktion, welche Verdampfungsrate mit Konzentration unter Gleichgewichtsbedingungen verknüpft, und einer zweiten linearen Regressionsfunktion, die Pseudogleichgewichtsverdampfungsraten in Flüchtigkeit umwandelt, und
- ein Mittel (330) zum Speichern der berechneten Verdunstungsrate und der berechneten Flüchtigkeit in einer Datenbank.

13. Vorhersagesystem für die Vorhersage der Entwicklung der physikalischen Parameter (400) eines Duftstoffs, um in Echtzeit prädiktive Kennzahlen zur Duftleistung bereitzustellen, **dadurch gekennzeichnet, dass** es umfasst:

- ein Mittel (405) zum Auswählen mindestens einer Kennung einer chemischen Verbindung in einer computerisierten Schnittstelle,
- ein Mittel (410) zum Eingeben einer Menge der chemischen Verbindung für jede ausgewählte chemische Verbindung,
- ein Mittel (415) zum Modellieren einer Ablagerung der Menge jeder ausgewählten chemischen Verbindung auf einer virtuellen Oberfläche durch Einstellen mindestens eines Parameters des Modells,
- ein Mittel (420) zum Simulieren der abgestreiften Menge der chemischen Verbindung in einem Luftstrom für jede modellierte abgelagerte chemische Verbindung durch ein Computersystem für mindestens zwei verschiedene Zeitpunkte als eine Funktion von:

- der Menge jeder der chemischen Verbindungen,
- einem ersten Wert, welcher eine virtuelle Oberflächengröße der abgelagerten chemischen Verbindung darstellt,
- einem zweiten Wert, der einen virtuellen Luftstrom darstellt, welcher auf die abgelagerte chemische Verbindung gerichtet ist und dazu konfiguriert ist, die chemische Verbindung virtuell von der Oberfläche abzustreifen,
- einem dritten Wert, welcher einen Aktivitätskoeffizienten jeder der chemischen Verbindungen darstellt, und
- einer mit der chemischen Verbindung verbundenen Flüchtigkeit, welche in einer Datenbank gespeichert ist, welche gemäß dem Datenbankaufbauverfahren nach einem der Ansprüche 1 bis 3 erstellt wurde, und

- ein Mittel (425) zum Anzeigen eines Indikators für jede chemische Verbindung, welcher die berechnete verdampfte Masse der Verbindung im Laufe der Zeit darstellt.

## Revendications

1. Procédé de construction de base de données de paramètres physiques de composé chimique liquide volatil (100), **caractérisé en ce qu'**il comprend :

- une étape (105) de dépôt commandé d'un composé chimique dans un récipient inerte, une quantité prédéterminée du composé chimique s'étalant sur une surface prédéterminée du récipient,
- une étape (110) de génération de flux d'air, le flux d'air étant dirigé dans la direction du composé chimique déposé,
- une étape (115) de mesure d'une quantité de composé chimique évaporé à différents moments de mesure,
- une étape de mesure de concentrations relatives en phase gazeuse pour plusieurs concentrations initiales d'ingrédients dans le composé chimique liquide dans des conditions de pseudo-équilibre expérimentales prédéfinies correspondant à des conditions d'équilibre,
- une étape (120) de calcul du taux d'évaporation en fonction des quantités de composés chimiques évaporés mesurées dans des conditions de pseudo-équilibre,
- une étape (125) de calcul de volatilité en fonction du taux d'évaporation calculé, une première fonction de régression linéaire reliant le taux d'évaporation à la concentration à des conditions d'équilibre et une seconde fonction de régression linéaire qui convertit des taux d'évaporation de pseudo-équilibre en volatilité et
- une étape (130) de stockage, dans une base de données, du taux d'évaporation calculé et de la volatilité calculée.

2. Procédé de construction (100) selon la revendication 1, qui comprend en outre :

- une étape (135) de calcul d'au moins une concentration en phase gazeuse du composé chimique pour une volatilité donnée du composé chimique,
- une étape (140) de mesure de l'intensité psychophysique du composé chimique pour au moins une dite concentration en phase gazeuse,
- une étape (145) de modélisation d'une formule mathématique d'intensité psychophysique du composé chimique en fonction de la concentration en phase gazeuse basée sur la base d'au moins deux des valeurs de concentration en phase gazeuse mesurées appelées « courbe dose-réponse » et
- une étape (150) d'enregistrement, dans une base de données, des paramètres modélisés par une formule d'intensité psychophysique du composé chimique.

3. Procédé de construction (100) selon la revendication 1 ou 2, qui comprend une pluralité d'étapes (105) de dépôt commandé d'un composé chimique à différentes températures, le taux d'évaporation étant calculé pour chaque dite température et stocké pendant l'étape de stockage.

**4.** Procédé de prédiction d'évolution de composition physique de parfum (200) pour fournir des mesures prédictives et en temps réel de la performance d'un parfum, **caractérisé en ce qu'**il comprend :

- une étape (205) de sélection d'au moins un identifiant de composé chimique dans une interface informatisée,
- une étape (210) de saisie, pour chaque composé chimique sélectionné, d'une quantité dudit composé chimique,
- une étape (215) de modélisation d'un dépôt de la quantité de chaque composé chimique sélectionné sur une surface virtuelle en réglant au moins un paramètre du modèle,
- une étape (220) de simulation, par un système informatique, pour chaque composé chimique déposé modélisé, de la quantité dépouillée dudit composé chimique dans un flux d'air pendant au moins deux moments différents en fonction :

  - de la quantité de chaque dit composé chimique,
  - d'une première valeur représentative d'une taille de surface virtuelle du composé chimique déposé,
  - d'une deuxième valeur représentative d'un flux d'air virtuel dirigé vers le composé chimique déposé configuré pour dépouiller virtuellement le composé chimique de la surface,
  - d'une troisième valeur représentative d'un coefficient d'activité de chaque dit composé chimique et
  - d'une volatilité associée audit composé chimique stockée dans une base de données construite selon le procédé de construction de base de données selon l'une quelconque des revendications 1 à 3 et

- une étape (225) d'affichage, pour chaque composé chimique, d'un indicateur représentatif de la masse évaporée calculée dudit composé au fil du temps.

**5.** Procédé de prédiction (200) selon la revendication 4, comprenant en outre une étape (230) de calcul d'une concentration en phase gazeuse du composé chimique virtuellement dépouillé en fonction de la quantité dépouillée calculée, l'étape (225) d'affichage étant configurée pour afficher la concentration en phase gazeuse calculée.

**6.** Procédé de prédiction (200) selon la revendication 5 lorsque la revendication 4 dépend de la revendication 2, comprenant en outre une étape (235) de calcul d'une intensité psychophysique de chaque composé chimique sélectionné en fonction de la concentration en phase gazeuse calculée, l'étape (225) d'affichage étant configurée pour afficher l'intensité psychophysique calculée au fil du temps.

**7.** Procédé de prédiction (200) selon la revendication 6, dans lequel au moins deux composés chimiques sont sélectionnés, le procédé comprenant en outre une étape (240) de calcul d'une intensité psychophysique globale comprenant :

- une première étape (241) de correspondance d'une valeur de concentration pour chaque composé à la courbe dose-réponse correspondante pour fournir une valeur d'intensité psychophysique pour ce composé,
- une deuxième étape (242) de correspondance de chaque dite valeur d'intensité psychophysique à une courbe dose-réponse fictive pour fournir une valeur de concentration de composé artificiel,
- une étape (243) d'ajout de chaque valeur de concentration de composé artificiel pour former une valeur de concentration virtuelle et
- une troisième étape (244) de correspondance de la valeur de concentration virtuelle à la courbe dose-réponse fictive pour fournir une valeur d'intensité psychophysique globale pour la composition.

l'étape d'affichage étant configurée pour afficher l'intensité psychophysique globale calculée au fil du temps.

**8.** Procédé de prédiction (200) selon la revendication 6 ou 7, dans lequel au moins deux composés chimiques sont sélectionnés, le procédé comprenant en outre une étape (245) de calcul d'une linéarité d'intensité psychophysique de la composition desdits au moins deux composés sur la base de l'intensité psychophysique calculée de chaque composé chimique sélectionné au fil du temps, l'étape (225) d'affichage étant configurée pour afficher la linéarité d'intensité psychophysique de la composition desdits au moins deux composés.

**9.** Procédé de prédiction (200) selon l'une quelconque des revendications 6 à 8, qui comprend en outre une étape (250) de sélection d'identifiant de composé chimique, ledit identifiant de composé chimique étant sélectionné si l'intensité psychophysique à un moment donné est inférieure à une valeur déterminée et une étape (255) d'affichage dudit identifiant de composé chimique.

**10.** Procédé de prédiction (200) selon l'une quelconque des revendications 4 à 9, dans lequel au moins deux composés chimiques sont sélectionnés pour former une composition, le procédé comprenant en outre une étape (260) de calcul de l'évolution de la composition au fil du temps en fonction de la quantité dépouillée calculée au fil du temps.

11. Procédé de prédiction (200) selon l'une quelconque des revendications 4 à 9, qui comprend en outre une étape de construction de la base de données de paramètres physiques de composé chimique liquide stockant la volatilité associée audit composé chimique en mettant en œuvre le procédé des revendications 1 à 3.

12. Système de construction de base de données de paramètres physiques de composé chimique liquide (300), **caractérisé en ce qu'**il comprend :

   - un moyen (305) de dépôt commandé d'un composé chimique dans un récipient inerte (306),
   - un moyen (310) de génération de flux d'air, le flux d'air étant dirigé dans la direction du composé chimique déposé,
   - un moyen (315) de mesure d'une quantité de composé chimique évaporé à différents moments de mesure,
   - un moyen de mesure de concentrations relatives en phase gazeuse pour plusieurs concentrations initiales d'ingrédients dans le composé chimique liquide dans des conditions expérimentales de pseudo-équilibre prédéfinies correspondant à des conditions d'équilibre,
   - un moyen (320) de calcul du taux d'évaporation en fonction des quantités de composés chimiques évaporés mesurées dans des conditions de pseudo-équilibre,
   - un moyen (325) de calcul de volatilité en fonction du taux d'évaporation calculé, une première fonction de régression linéaire reliant le taux d'évaporation à la concentration à des conditions d'équilibre et une seconde fonction de régression linéaire qui convertit les taux d'évaporation de pseudo-équilibre en volatilité et
   - un moyen (330) de stockage, dans une base de données, du taux d'évaporation calculé et de la volatilité calculée.

13. Système de prédiction d'évolution de paramètres physiques de parfum (400) pour fournir des mesures prédictives et en temps réel des performances d'un parfum, **caractérisé en ce qu'**il comprend :

   - un moyen (405) de sélection d'au moins un identifiant de composé chimique dans une interface informatisée,
   - un moyen (410) de saisie, pour chaque composé chimique sélectionné, d'une quantité dudit composé chimique,
   - un moyen (415) de modélisation d'un dépôt de la quantité de chaque composé chimique sélectionné sur une surface virtuelle en réglant au moins un paramètre du modèle,
   - un moyen (420) de simulation, par un système informatique, pour chaque composé chimique déposé modélisé, de la quantité dépouillée dudit composé chimique dans un flux d'air pendant au moins deux moments différents en fonction :

      - de la quantité de chaque dit composé chimique,
      - d'une première valeur représentative d'une taille de surface virtuelle du composé chimique déposé,
      - d'une deuxième valeur représentative d'un flux d'air virtuel dirigé vers le composé chimique déposé configuré pour dépouiller virtuellement le composé chimique de la surface,
      - d'une troisième valeur représentative d'un coefficient d'activité de chaque dit composé chimique et
      - d'une volatilité associée audit composé chimique stockée dans une base de données construite selon le procédé de construction de base de données selon l'une quelconque des revendications 1 à 3 et

   - un moyen (425) d'affichage, pour chaque composé chimique, d'un indicateur représentatif de la masse évaporée calculée dudit composé au fil du temps.

Figure 1

100

| | |
|---|---|
| 105 | Controlled deposition |
| 110 | Airflow generation |
| 115 | Measurement of the quantity of evaporated compound |
| 120 | Evaporation rate calculation |
| 125 | Volatility calculation |
| 130 | Storing |
| 135 | Computing gas phase concentration |
| 140 | Measurement of the psychophysical intensity |
| 145 | Modeling |
| 150 | Modeling |

Figure 2

200

| 100 | Compound physical parameter database construction |
|---|---|
| 205 | Selecting at least one compound |
| 210 | Inputting a quantity |
| 215 | Modeling a deposition |
| 220 | Simulating |
| 230 | Calculating a gas phase concentration |
| 235 | Computing a psychophysical intensity |

| 240 | Computing a global psychophysical intensity |
|---|---|
| 241 | First matching |
| 242 | Second matching |
| 243 | Addition |
| 244 | Third matching |

| 245 | Computing a global psychophysical intensity linearity |
|---|---|
| 260 | Computing a composition evolution |
| 225 | Displaying |
| 250 | Chemical compound identifier selection |
| 255 | Display |

Figure 3

Figure 4

400

430

405 410 415 420 425

Figure 5

Figure 6

Figure 7

810

815

805

Figure 8

910

915

905

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019238680 A

- SU 1013822

**Non-patent literature cited in the description**

- **PENPAN SAIVASOMBATI et al.** *Disposition of Benzyl Alcohol After Topical Application to Human Skin in vitro*

- **G.B. KASTING et al.** *A physico-chemical properties based model for estimating evaporation and absorption rated of perfumes from skin*